# EUROPEAN PATENT SPECIFICATION

(11) **EP 3 973 868 B1**
(45) Date of publication and mention of the grant of the patent: **12.03.2025**
(21) Application number: 21199445.4
(22) Date of filing: 28.09.2021
(51) Int. Cl.: A61B 5/11, A61B 5/024

(54) **SAFETY BAND APPLICABLE TO CLASPS OF WATCHES AND BRACELETS IN GENERAL AND INTEGRATING A SYSTEM FOR DETECTING POSITION AND/OR HEARTBEAT**
SICHERHEITSBAND, DAS FÜR SCHLIESSEN VON UHREN UND ARMBÄNDER IM ALLGEMEINEN ANWENDBAR IST UND EIN SYSTEM ZUR ERKENNUNG DER POSITION UND/ODER DES HERZSCHLAGS INTEGRIERT
BRACELET DE SÉCURITÉ APPLICABLE AUX FERMOIRS DE MONTRES ET BRACELETS EN GÉNÉRAL ET INTEGRANT UN SYSTÈME DE DÉTECTION DE POSITION ET/OU DE BATTEMENT CARDIAQUE

(30) Priority: 28.09.2020 IT 202000022774
(43) Date of publication of application: 30.03.2022
(73) Proprietor: Polso S.r.l., 50132 Firenze (IT)
(72) Inventor: ANDORLINI, Tommaso Maria, 50132 Firenze (IT); MOSCARDINI, Sara, 50132 Firenze (IT)
(74) Representative: Emmi, Mario

(56) References cited:
- KR-A- 20160 007 020
- KR-U- 20120 008 484
- US-A1- 2016 106 333
- US-A1- 2017 031 326

## Description

### Field of the invention

The present invention concerns the technical field of wrist object accessories.

In particular, the disclosure relates to an innovative device which enables the user to use his wristwatch, as well as other wrist objects, as a detecting system of his physical activity, such as the detecting of the heart rate frequency and/or even for taking the user's geo-localisation, all that by enhancing security for avoiding the inadvertent misplacing of one's own watch or the wrist object in general.

### Overview of the prior art

Nowadays, there are several sport wrist products, for example heart rate monitors, GPS tracker, etc. Some of these products are real wrist computers, specifically programmed for giving support to the sportsman in his work out.

Thanks to the functions of geo-localisation, heart rate frequency measurement, etc., the user can carry out targeted and precise trainings by keeping precise regularities depending on the type of required exercise. In certain cases, for example, the sportsman must carry out targeted trainings at a specific heart rate frequency or he must exercise himself at specific regularities. Even in everyday life, a monitor of this kind can help to control and improve health, by monitoring the basal metabolism, the calorie consumption, the sleep schedules and the immune system.

Therefore, there are several products on the market that can satisfy a vast range of demands.

In the light of the above, it is clear that, for taking advantage from these functions, the user is obliged to buy a specific product, and therefore he is obliged to face an expense.

Although the enthusiasts and the agonists face the expense without problems and, rather, they are searching for specific products, a vast range of public, which are not agonists but purely amateurs and/or occasionals, choose more simple products. Heart rate monitor watches, for example, fall within these simple products. This range of public, being without specific agonistic aims, often gives up the purchase of even simple and low-cost products, and merely limit themselves to use their own everyday wristwatch which, though, does not have the function of heart rate monitor or other simple functions.

Moreover, not least, the use of non-specific watches has the technical disadvantage that these are not specifically suitable for stresses, and therefore while practicing sport, for example running, they might also detach inadvertently, and the user might lose them inadvertently. Anyway, the same happens with the traditional bracelets that the user may usually wear at his wrist.

Publications US2017/031326, US2016/106333, KR20210008484, KR20160007020 are also known.

### Summary of the invention

Hence, it is the aim to provide an accessory for wrist objects that solves the said technical disadvantages.

In particular, it is the aim tc provide a device which can be applied, as an accessory, to the fastening of a pre-existing wrist object, for example a watch or a bracelet, for the purpose of increasing the safety of the fastening itself, and, at the same time, fulfilling and integrating certain technical functions which are specific of certain devices, such as the detecting of heart rate frequency or even so the detection of parameters related to the user's physical activity (also a possible geo-localisation).

Said device is in the form of a strap foldable on itself. Therefore, the strap comprises quick attachment means for enabling the closing on itself, and therefore fixing said strap around an object, such as a bracelet or a watch.

For example, such quick attachment means can be in the form of Velcro or pressure buttons with snap-like lock, etc.

Moreover, said strap integrates means (40) configured to detect one or more parameters related to the user's ongoing physical activity, for example the detecting of heart rate frequency or a geo-localisation or both of them.

In this way, all the said technical disadvantages are solved easily.

Particularly, by simply applying said strap around the fastening of one's own watch or bracelet, on the one hand the safety for avoiding the inadvertently loss of the watch is increased, and, on the other hand, certain functions are integrated without the need of buying a specific watch.

The data detected by said means are sent to an external device, such as a mobile phone device preferably, for example a smartphone or an Apple (IOS) or Android device. The external device can also be, alternatively, a computer, for example on the laptop type.

In any case, the connection between the external device and said strap can be carried out wirelessly, such as via *Bluetooth,* and for example through the installation of its downloadable application (this application can be made available on suitable platforms when it is launched).

The strap, by closing on itself around the fastening of the watch or the wrist object in general, prevents it from detaching inadvertently, and therefore it is a safety against the inadvertent loss of the wrist object to which the user can be affectively attached, and which can obviously have even an economic value.

The means for detecting one or more parameters linked to the physical activity done by the user, for example the possible heart rate frequency detecting sensor, enable the user to receive information otherwise obtainable from specific watches, for example the heart rate, as well as the possible geo-localisation and/or the basal metabolism.

The strap, therefore, is connected wirelessly to the external device, for example the user's mobile phone device, and thus the strap and the device begin to communicate.

The user can, therefore, download a specific App, and therefore he can easily monitor directly on the mobile device any of the detected measurements.

The strap detects the parameters and transmits them to the electronic external device, which displays them.

In this way, all the said technical disadvantages are solved.

In particular, the user can now buy this accessory which is applied around his watch belt, in particular, around its fastening.

The user is no longer obliged to buy a new watch and he will have the opportunity to use his customary watch and, thanks to the device, which is the subject of this invention, he will be able to integrate specific functions, such as the heart rate detection.

The device is compatible and connects to electronical external devices, for example PCs, data processors in general and mobile phone devices preferably.

The communication with the mobile phone device, for example through a specific "App", is a preferred solution given that the mobile phone device is a device of common and daily use.

In this way, in real time, the user can view on his external device the measured data and such data can also be memorized in a log file history.

Through a specific app, therefore, the user creates his own private area where the data are memorized, and thus the user can view the whole history.

Advantageously, such means can comprise
- Measurement means for detecting at least one user's physiological parameter, for example the heart rate frequency and/or;
- Geo-localisation means configured to detect the user's position.

In this way, the detected physiological parameters can be correlated to data of routes and distances travelled.

In a variant, only geo-localisation means can be present without the detection means. In this solution, the user does not display his own one or more vital parameters, but he has a geo-localisation function anyway.

Therefore, in accordance with the invention, there is also here described the use of a
device, in accordance with what described above, for being applied around the fastening of a wrist object to which it is intended so as to have, contextually, the function of safety fastening and detecting at least one measurement related to the activity carried out by the user (therefore, the detection of one or more physiological parameters, for example heart rate, and/or geo-localisation).

There is also here described a method for taking at least one measurement related to the physical activity performed by a person, the method providing the application of a device, in accordance with what described above, around the fastening of a wrist object worn by the user, and the activation of said device which detects the measurement and communicates with an external electronic device through which the measures taken by said device are displayed.

There is also described here a system comprising:
- A device in the form of a strap in accordance with one or more of the features described above;
- An external electronic device, for example a mobile phone device, communicating, preferably wirelessly, with the said device in the form of a strap.

Further advantages can be inferred from the dependent claims.

### Description of the drawings in brief

Further features and the advantages of this device
will result clearer with the following description of some of its embodiments, made as an example and non-limiting, referring to the enclosed drawings, wherein:
- Figure 1 shows a plan view of the strap formed by three panels (A,B,C) foldable on each other through fold lines;
- Figure 2 shows the plan view from the opposite side and the sensor for detecting the frequency is highlighted by the dotted line;
- Figure 3 schematises one application onto a watch;
- Figure 4A schematises the device in communication with a mobile phone device 100 through which the data detected by the said device are displayed and/or processed and/or memorized.
- Figure 4B shows, with a simple flow chart, that the data can be processed in the processor of the mobile phone device directly, so that they can be memorized and/or processed and/or displayed, once they have been acquired since they are transmitted by the strap-form device.

### Description of some preferred embodiments

It is here described an accessory applicable on wrist objects, such as watches and bracelets in general.

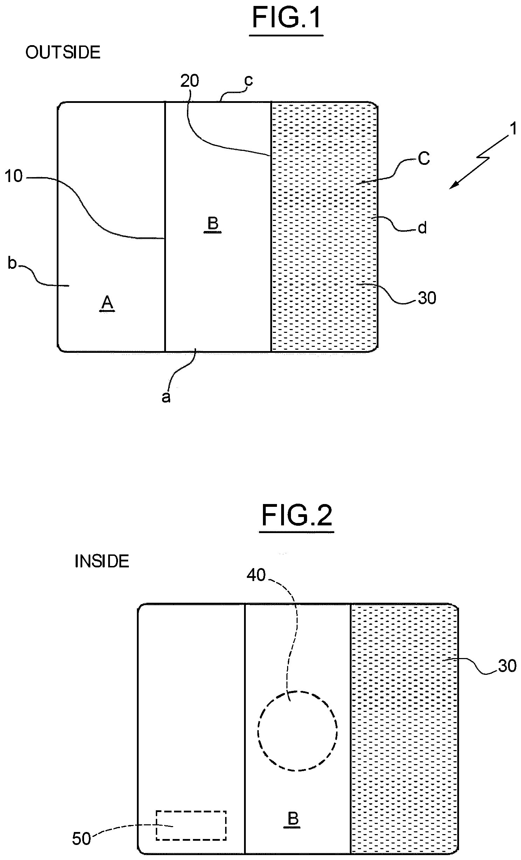

Figure 1 shows the accessory, which is in the form of a strap 1 that can be closed on itself.

The strap is preferably rectangular or generally rectangular, even if, for example, a squared or generally squared form could equally be possible.

The strap 1 is formed by a certain number of panels linked to each other and foldable on each other.

In particular, the strap 1 is formed by three panels (A, B, C) highlighted in figure 1. For this aim, therefore, two lines (10, 20) parallel to each other are provided within the area of the strap and representing the fold lines.

More particularly, the strap is formed by four perimetral sides, or rather two longitudinal sides (a, c) parallel to each other and two vertical sides (b, d) parallel to each other.

In the case of a rectangular form (or substantially rectangular), such four sides are connected in succession (with sharp or rounded corners), so as to delimit the rectangle formed by the two vertical sides with an inferior length with respect to the two longitudinal sides.

The opposite, wherein the two vertical sides are longer than the longitudinal ones or, in the case of square form, with all of the four sides with the same detection, could be possible.

The parallel fold lines (10, 20) are, therefore, two vertical lines parallel to each other and parallel to the vertical sides (b, d). Said fold lines 10 and 20 extend within the strap area, starting from one longitudinal side (a, c) connecting to the opposite longitudinal side, and therefore extending vertically, with respect to the longitudinal sides, within the strap area.

Such fold lines 10 and 20 delimit, therefore, the three panels (A, B, C) and are such as to enable a panel to rotate around this line thereby overlapping the other panel.

A fold line can, for example, be made in the form of an incision into the material, or anyway by making a line along which the thickness has been reduced.

Panels detached and connected by hinge systems could equally be possible.

In any case, in this way each panel can rotate around the axis defined by such fold line.

Figure 1 shows, therefore, a face of the strap 1 while figure 2 shows the opposite face and highlights, in correspondence of the central panel B, a site 40 where the electronical components are inserted, for example embedded, for fulfilling the needed technical functions, or rather the means for measuring a parameter linked to the user's physical activity, for example physiological parameters, such as heart rate frequency and/or basal metabolism and/or geo-localisation.

Specifically, it is possible to provide means comprising:
- Measuring means to detect at least one physiological parameter of the user, for example the heart rate frequency;
- Geo-localisation means configured to detect the user's position.

In the light of the above, a heart rate frequency monitor device and/or a GPS tracker, for example, can be inserted into said site 40.

Obviously, other devices, and therefore other functions, could be inserted, and therefore the ones mentioned above (heart rate frequency and/or geo-localisation) are the preferred ones, but they could also be integrated or replaced by other ones.

Therefore, it is possible to have a device detecting only the user's physical parameters (one or more than one), such as heart rate frequency.

A device detecting only the position.

A device detecting the position and one or more physical parameters.

In correspondence of panel (A) it is instead provided a site 50 for housing a battery, possibly rechargeable, possibly integrable into the panel B itself.

Panel C comprises first quick attachment means which engage with second quick attachment means provided in panel A so that these can engage reciprocally.

Such quick attachment means can provide a Velcro system, as well as quick pressure systems, such as snap-like buttons or connections in general of magnetic type.

In the case of Velcro, but the same is valid for the other quick systems useable, a part of the Velcro is applied on the face of panel C, while the counterposed Velcro is applied on the opposite face of panel A. In this way, when panel A is folded onto the central panel B by rotating around the fold line 10 (or vice versa), the Velcro of panel A is facing the Velcro of panel C, when this is rotated onto panel A.

In this way, by folding panel C toward panel B, through a rotation around the fold line 20, it is possible for the Velcro of panel C to be engaged with the corresponding Velcro of panel A, and with the central panel B, included between A and C.

The same happens in the case of snap-like and pressure buttons or magnetic systems.

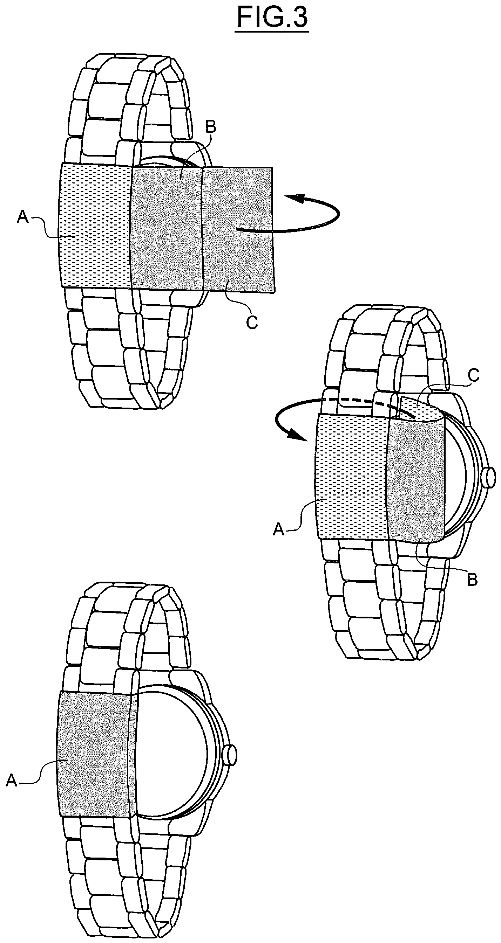

Figure 3 shows an application of the described strap to a watch, in particular, right around the fastening of the watch itself, so as to surround said fastening.

Panel A is put in touch on the fastening area of the watch, with the Velcro (represented with a dotted area) facing toward the outside (that is, from the opposite side of the fastening).

Figure 3, therefore, shows the outstretched strap, that is open in the form of a plane, wherein panel A, at the moment of the application, must be positioned in correspondence of the watch's fastening on the external part, that is on the fastening part which is not facing toward the user's wrist and with the Velcro (or another above-introduced equal system) therefore facing toward the outside. Then, B is folded toward A making B pass under the wrist, thereby positioning panel B in correspondence of the fastening on the side facing, in use, toward the wrist in overlapped position to A, so that the watch fastening is included between the two panels A and B.

Then, C is also rotated against A, so that the quick attachment means (in this case the Velcro, for example) grip on each other (C overlapping A).

In this way, there is the double effect of avoiding an inadvertent opening of the watch fastening, because this is closed by the strap device subject of the invention. Moreover, the system detects parameters and performs functions depending on the components integrated into it, and therefore through the means integrated into it.

By manufacturing the strap device with a suitable longitudinal length and elastic compliance, indeed, it is possible to make it well tighten around the fastening of the wrist object, to which it is applied, thereby exerting the function of inadvertent anti-unhook safely.

The central panel B is the one including into it the electronic parts (that is the means) for carring out the desired functions, for example the detection of heart rate frequency and/or basal metabolism and/or a geo-localisation, and it must be positioned, therefore, so as to be in touch with the user's wrist, in use.

For this aim, therefore, the described embodiment in the form of three panels (A, B, C) foldable on each other is convenient because it eases the application on the wrist, and besides it obliges a suitable positioning so that panel B results in touch with, or anyway well in correspondence of the wrist.

The manufacturing material is preferably a highly flexible and soft material, such as rubber, so as to be able to flex for the application easily and to be comfortable.

As it is shown in figure 2, indeed, the site 40 includes means for detecting a measurement of the user's physical activity, for example the measure of a physiological parameter and/or a geo-localisation.

In particular, measuring means can be provided for detecting a measurement of a physiological parameter of the user (for example the heart rate frequency).

These can engage with geo-localisation means for detecting a position.

The measuring means are in touch with the wrist when the described application is carried out so as to be able to monitor the various vital parameters detectable through the wrist with the well-known technology (for example, indeed, the detecting of heart rate frequency).

A wide range of materials can be selected for manufacturing the described strap.

For example, it is possible to manufacture the whole with a material of a gummy nature (for example plastic) with a specific stiffness and including inside it the requested electronic parts. Other stiff materials can be used.

Alternatively, the site 40 could be openable and closable with a small door for easing the access to the internal electronic parts.

Obviously, the strap could anyway be wholly manufactured in a very soft and flexible material, such that it does not need the fold lines, but, in that case, the user should be more careful to apply it correctly to the wrist.

In all the described cases, the preferred manufacturing material is of the waterproof type, therefore water resistant, such as a material like rubber etc. and the site 40 is waterproof to avoid infiltrations of liquids because of, for example, sweating.

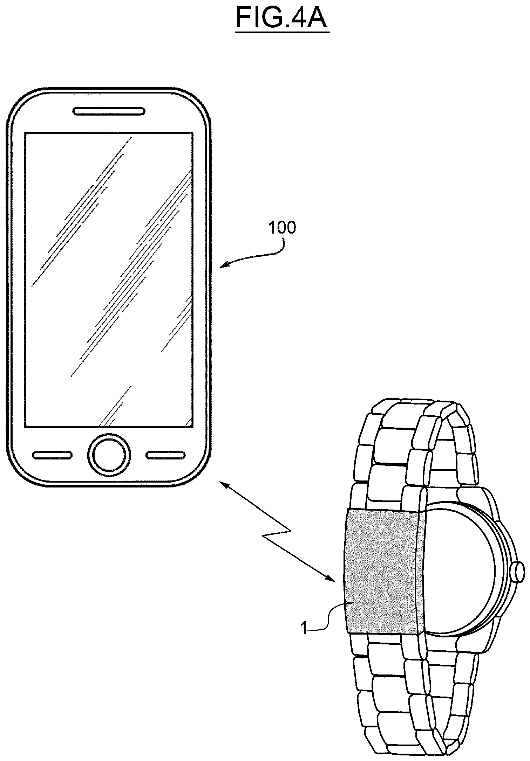

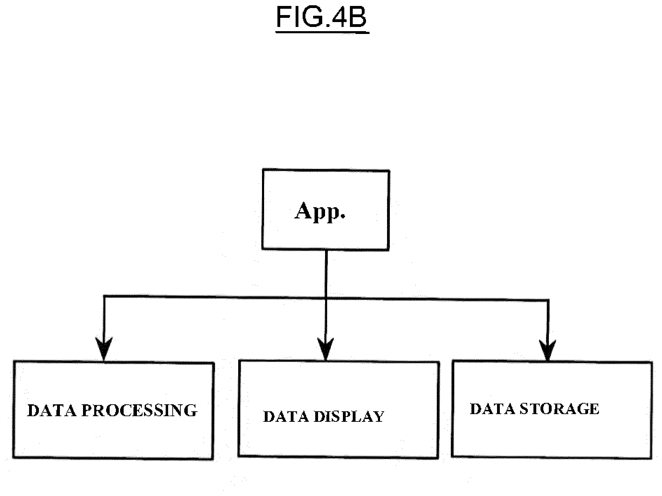

Therefore, as shown in figure 4A, a mobile phone device 100 communicating with the strap is represented, so that the user, in real time, can read the detected data.

The mobile phone device can be substituted by any other electronic device equipped with a display for displaying data (for example a PC).

In the case of a mobile phone, it is possible to provide a specific "App." downloadable on the mobile phone device which, after the user's registration, identifies the specific user's strap to which it connects. The user can, therefore, monitor his own data which can also be memorized and displayed later.

The device 1 can, therefore, be equipped with a processor that processes data as well as it could only detect the measurements to then send them to the App, which processes and/or memorizes them later for its displaying even in real time.

The sensors integrable into the device 1 are, therefore, the well-known ones and already integrated into specific devices such as:
- Heart rate frequency detection;
- Pressure;
- Geo-localization.

One or more of these ones are present and further parameters could be easily integrated.

As it is already known in the technical field of the sport devices, data can be memorized and then displayed in many forms, so that the user can verify a history of the results.

## Claims

1. The use of a device to be applied around the fastening of a wrist object, such as a watch or a bracelet, to which it is intended so that the said device, by closing
on itself around the fastening of the said wrist object, contextually has a function of safety fastening against the inadvertent loss of the said wrist object and detecting of at least one measurement related to the physical activity performed by the user, the said device being in the form of a strap (1) configured to be roll-up on itself, said strap comprising quick attachment means (30) for fixing themselves in a releasable manner to the winding position on itself around the fastening of the said wrist object and further comprising:
- Means (40) configured to detect at least one measurement related to the user;
- The device being communicable with an external electronic device (100), preferably a mobile telephone device (100), through which the said at least one detected measurement can be displayed.

2. The use, according to claim 1, wherein said means comprise:
- Measuring means to detect at least one physiological parameter of the user, for example the heart rate and/or;
- Geo-localization means configured to detect the user's position.

3. The use, according to claim 2, wherein said measuring means comprise at least one heart rate monitor so that said measurement comprises at least the detection of the heart rate.

4. The use, according to one or more of the preceding claims, wherein said strap is formed by at least three panels (A, B, C) foldable over each other through a fold line.

5. The use, according to claim 4, wherein the strap is formed by four sides (a, b, c, d) which delimit perimetrically the strap and of which two longitudinal sides (a, c) and two vertical sides (b, d), said fold lines being two vertical fold lines (10, 20) parallel to the perimetral vertical lines (b, d) and which starting from a longitudinal perimetral side (a) join the opposite longitudinal side (c), said two vertical fold lines developing within the area of the strap each one at a certain distance from a perimetral vertical side so as to form the said at least three panels (A, B, C).

6. The use, according to one or more of the preceding claims, wherein the manufacturing material is water-repellent.

7. The use, according to one or more of the preceding claims, wherein said quick attachment means optionally include:
- A Velcro;
- Quick snap-like means;
- Magnets.

8. The use according to claims 4 or 5, wherein the said fold line is in the form of an incision into the thickness of the strap.

## Patentansprüche

1. Verwendung einer Vorrichtung, die um die Befestigung eines Gegenstandes am Handgelenk, wie z.B. einer Uhr oder eines Armbandes, angebracht werden soll, so dass die Vorrichtung, indem sie sich um die Befestigung des Gegenstandes am Handgelenk schließt, kontextuell eine Funktion der Sicherheitsbefestigung gegen den unbeabsichtigten Verlust des Gegenstandes am Handgelenk und der Erfassung von mindestens einer Messung in Bezug auf die vom Benutzer ausgeübte körperliche Aktivität aufweist, wobei die Vorrichtung die Form eines Bandes (1) aufweist, das so konfiguriert ist, dass es um sich selbst aufgerollt werden kann, wobei das Band Schnellanbringungsmittel (30) umfasst, um sich selbst in lösbarer Weise an der Aufwickelposition um die Befestigung des Gegenstands am Handgelenk zu fixieren, und zudem umfasst:
- Mittel (40), die so konfiguriert sind, dass sie mindestens eine auf den Benutzer bezogene Messung erfassen;
- Die Vorrichtung ist mit einem externen elektronischen Gerät (100), vorzugsweise einem Mobiltelefon (100), kommunizierbar, über das die mindestens eine erfasste Messung angezeigt werden kann.

2. Verwendung nach Anspruch 1, wobei die Mittel Folgendes umfassen:
- Messmittel zur Erfassung mindestens eines physiologischen Parameters des Benutzers, z. B. der Herzfrequenz und/oder;
- Geolokalisierungsmittel, die so konfiguriert sind, dass sie die Position des Benutzers erkennen.

3. Verwendung nach Anspruch 2, wobei die Messmittel mindestens einen Herzfrequenzmesser umfassen, so dass die Messung mindestens die Erfassung der Herzfrequenz umfasst.

4. Verwendung nach einem oder mehreren der vorhergehenden Ansprüche, **dadurch gekennzeichnet, dass** das Band aus mindestens drei Paneelen (A, B, C) gebildet wird, die durch eine Faltlinie übereinander faltbar sind.

5. Verwendung nach Anspruch 4, **dadurch gekennzeichnet, dass** das Band durch vier Seiten (a, b, c, d) gebildet wird, die das Band umfangsmäßig begrenzen und von denen zwei Längsseiten (a, c) und zwei vertikale Seiten (b, d) sind, wobei die Faltlinien zwei vertikale Faltlinien (10, 20) parallel zu den vertikalen Umfangslinien (b, d) sind und die von einer Umfangslängsseite (a) ausgehend in die gegenüberliegende Längsseite (c) übergehen, wobei die zwei vertikalen Faltlinien im Bereich des Bandes jeweils in einem bestimmten Abstand von einer vertikalen Umfangsseite verlaufen, so dass die mindestens drei Paneelen (A, B, C) gebildet werden.

6. Verwendung nach einem oder mehreren der vorhergehenden Ansprüche, wobei das Herstellungsmaterial wasserabweisend ist.

7. Verwendung nach einem oder mehreren der vorangehenden Ansprüche, wobei die Schnellanbringungsmittel optional umfassen:
- Ein Klettverschluss;
- Ein schnelles Schnappmittel;
- Magnete.

8. Verwendung nach Anspruch 4 oder 5, **dadurch gekennzeichnet, dass** die Faltlinie in Form eines Einschnitts in die Dicke des Bandes ausgebildet ist.

## Revendications

1. Utilisation d'un dispositif à appliquer autour de la fixation d'un objet de poignet, tel qu'une montre ou un bracelet, auquel il est destiné afin que ledit dispositif, en se fermant sur lui-même autour de la fixation dudit objet de poignet, ait contextuellement une fonction de fixation de sécurité contre la perte involontaire dudit objet de poignet et de détection d'au moins une mesure relative à l'activité physique exercée par l'utilisateur, ledit dispositif se présentant sous la forme d'une bande (1) configurée pour être enroulée sur elle-même, ladite bande comprenant un moyen d'attache rapide (30) pour se fixer de manière amovible à la position d'enroulement sur elle-même autour de la fixation dudit objet de poignet et comprenant en outre :
- un moyen (40) configuré pour détecter au moins une mesure relative à l'utilisateur ;
- le dispositif pouvant communiquer avec un dispositif électronique externe (100), de préférence un téléphone mobile (100), par lequel ladite au moins une mesure détectée peut être affichée.

2. Utilisation selon la revendication 1, ledit moyen comprenant :
- des moyens de mesure pour détecter au moins un paramètre physiologique de l'utilisateur, par exemple la fréquence cardiaque et/ou ;
- des moyens de géolocalisation configurés pour détecter la position de l'utilisateur.

3. Utilisation selon la revendication 2, lesdits moyens de mesure comprenant au moins un moniteur de fréquence cardiaque, de sorte que ladite mesure comprend au moins la détection de la fréquence cardiaque.

4. Utilisation selon l'une ou plusieurs des revendications précédentes, ladite bande étant formée d'au moins trois panneaux (A, B, C) pouvant être repliés les uns sur les autres par une ligne de pliage.

5. Utilisation selon la revendication 4, la bande étant formée de quatre côtés (a, b, c, d) qui délimitent de manière périphérique la bande et dont deux côtés longitudinaux (a, c) et deux côtés verticaux (b, d), lesdites lignes de pliage étant deux lignes de pliage verticales (10, 20) parallèles aux lignes verticales périphériques (b, d) et qui, partant d'un côté longitudinal périphérique (a) joignent le côté longitudinal opposé (c), lesdites deux lignes de pliage verticales se développant, dans la zone de la bande, chacune à une certaine distance d'un côté vertical périphérique de manière à former lesdits au moins trois panneaux (A, B, C).

6. Utilisation selon l'une ou plusieurs des revendications précédentes, le matériau de fabrication étant hydrofuge.

7. Utilisation selon l'une ou plusieurs des revendications précédentes, le moyen d'attache rapide comprenant éventuellement :
- un velcro ;
- un moyen à encliquetage rapide ;
- des aimants.

8. Utilisation selon les revendications 4 ou 5, ladite ligne de pliage se présentant sous la forme d'une incision dans l'épaisseur de la bande.
